# EUROPEAN PATENT APPLICATION

(11) **EP 3 005 985 A1**
(43) Date of publication of application: **13.04.2016**
(21) Application number: 15188492.1
(22) Date of filing: 06.10.2015
(51) Int. Cl.: A61F 2/24

(54) **BI-LEAFLET MITRAL VALVE DESIGN**

(30) Priority: 07.10.2014 US 201462060613 P
(71) Applicant: St. Jude Medical, Cardiology Division, Inc., St. Paul MN 55117 (US)
(72) Inventor: DALE, Theodore Paul, Corcoran, MN Minnesota 55340 (US); PARA, Andrea N., St. Louis, MO Missouri 63130 (US); DIEDERING, Jason, Minneapolis, MN Minnesota 55407 (US)
(74) Representative: Gill Jennings & Every LLP

(57) **Abstract**

A prosthetic mitral valve (300) includes a collapsible and expandable elliptical or "D"-shaped stent (350). The stent includes first and second commissure attachment features (316) and a collapsible and expandable valve assembly (360) disposed therein. The valve assembly includes two leaflets (362), each leaflet having a first edge (374) operably coupled to the stent and a second free edge (376). Each leaflet may include first and second tabs (370, 372) connecting first and second ends of the first and second edges, respectively. Each leaflet includes a height measured from a midpoint of the first edge to a midpoint of the second edge and a width measured from a junction of the first edge with the first tab to a junction of the first edge with the second tab. The ratio of the height to the width may be between about 0.4 and about 0.65.

## Description

### CROSS-REFERENCE TO RELATED APPLICATIONS

The present application claims the benefit of the filing date of U.S. Provisional Patent Application No. 62/060,613 filed October 7, 2014, the disclosure of which is hereby incorporated herein by reference.

### BACKGROUND OF THE DISCLOSURE

The present disclosure relates to prosthetic heart valves and, in particular, collapsible prosthetic mitral valves.

Prosthetic heart valves that are collapsible to a relatively small circumferential size can be delivered into a patient less invasively than valves that are not collapsible. For example, a collapsible valve may be delivered into a patient via a tube-like delivery apparatus such as a catheter, a trocar, a laparoscopic instrument, or the like. This collapsibility can avoid the need for a more invasive procedure such as full open-chest, open-heart surgery.

Collapsible prosthetic heart valves typically take the form of a valve structure mounted on a stent. There are two types of stents on which the valve structures are ordinarily mounted: a self-expanding stent and a balloon-expandable stent. To place such valves into a delivery apparatus and ultimately into a patient, the valve must first be collapsed or crimped to reduce its circumferential size.

When a collapsed prosthetic valve has reached the desired implant site in the patient (e.g., at or near the annulus of the patient's heart valve that is to be replaced by the prosthetic valve), the prosthetic valve can be deployed or released from the delivery apparatus and re-expanded to full operating size. For balloon-expandable valves, this generally involves releasing the entire valve, assuring its proper location, and then expanding a balloon positioned within the valve stent. For self-expanding valves, on the other hand, the stent automatically expands as the sheath covering the valve is withdrawn.

Prosthetic valves, particularly those for replacement of a native aortic valve, often contain three coapting leaflets as part of a valve assembly having a substantially circular or cylindrical shape, the valve assembly being supported by a substantially cylindrical stent. Although this type of prosthetic valve can be used to replace a native mitral valve, problems may arise from such implantation. For example, upon implantation into the native mitral valve annulus, a prosthetic heart valve with a cylindrical stent and cylindrical valve assembly having three leaflets may deform substantially to fit the elliptical geometry of the native mitral valve annulus. This deformation may prevent the three leaflets from properly coapting with one another to form a seal, which in turn may result in a greater degree of regurgitation *(i.e.,* retrograde blood flow through the prosthetic valve). For this and other reasons, it would be desirable to have a prosthetic mitral valve better suited to the architecture of the native mitral valve.

### BRIEF SUMMARY

In one embodiment, a prosthetic mitral valve includes a collapsible and expandable stent, and first and second commissure attachment features disposed on the stent. A collapsible and expandable valve assembly may also be disposed within the stent. The valve assembly may include two leaflets, each leaflet having a first edge operably coupled to the stent and a second free edge. Each leaflet may further include a first tab connecting a first end of the first edge to a first end of the second free edge, and a second tab connecting a second end of the first edge to a second end of the second free edge. Each leaflet may also include a height measured from a midpoint of the first edge to a midpoint of the second edge and a width measured from a junction of the first edge with the first tab to a junction of the first edge with the second tab. The ratio of the height to the width may be between about 0.4 and about 0.65.

In another embodiment, a prosthetic mitral valve includes a collapsible and expandable stent and first and second commissure attachment features disposed on the stent. A collapsible and expandable valve assembly may also be disposed within the stent. The valve assembly may include two leaflets, a first portion of each leaflet coupled to the first commissure attachment feature and a second portion of each leaflet coupled to the second commissure attachment feature. The stent in an expanded condition may be substantially elliptical in transverse cross-section and have a minor axis with a length and a major axis with a length. The ratio of the length of the minor axis to the length of the major axis may be between about 0.7 and about 0.99.

### BRIEF DESCRIPTION OF THE DRAWINGS

FIG. 1 is a schematic cut-away view of a heart.
FIG. 2 is a schematic view of a native mitral valve.
FIG. 3A is side view of a prosthetic mitral valve in an expanded condition according to an aspect of the disclosure.
FIG. 3B is a perspective view of the prosthetic mitral valve of FIG. 3A.
FIG. 4 is a longitudinal cross-section of the prosthetic mitral valve of FIG. 3A.
FIG. 5 is an end view of the stent of the prosthetic mitral valve of FIG. 3A in the expanded condition.
FIG. 6 is an end view of an alternate stent of a prosthetic mitral valve in the expanded condition.
FIG. 7 is a plan view of a leaflet of the prosthetic mitral valve of FIG. 3A.

### DETAILED DESCRIPTION

Blood flows through the mitral valve from the left atrium to the left ventricle. As used herein, the term "inflow," when used in connection with a prosthetic mitral valve, refers to the end of the heart valve closest to the left atrium when the heart valve is implanted in a patient, whereas the term "outflow," when used in connection with a prosthetic mitral valve, refers to the end of the heart valve closest to the left ventricle when the heart valve is implanted in a patient. As used herein, the terms "substantially," "generally," "approximately," and "about" are intended to mean that slight deviations from absolute are included within the scope of the term so modified. When ranges of values are described herein, those ranges are intended to include sub-ranges. For example, a recited range of 1 to 10 includes 2, 5, 7, and other single values, as well as ranges of 2 to 6, 3 to 9, 4 to 5, and others.

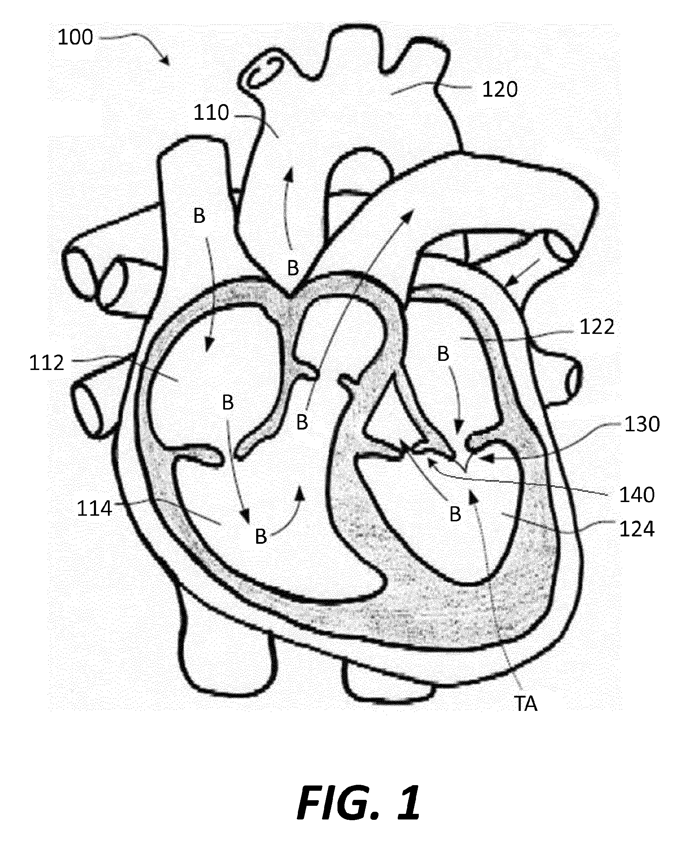

FIG. 1 is a schematic representation of a human heart 100. The human heart includes two atria and two ventricles: a right atrium 112 and a left atrium 122, and a right ventricle 114 and a left ventricle 124. As illustrated in FIG. 1, the heart 100 further includes an aorta 110, and an aortic arch 120. Disposed between the left atrium 122 and the left ventricle 124 is the mitral valve 130. The mitral valve 130, also known as the bicuspid valve or left atrioventricular valve, is a dual-flap valve that opens as a result of increased pressure from the left atrium 122 as it fills with blood. As atrial pressure increases above that of the left ventricle 124, the mitral valve 130 opens and blood passes toward the left ventricle. Similarly, disposed between aorta 110 and left ventricle 124 is the aortic valve 140. The aortic valve is a tricuspid valve that opens as a result of increased pressure from the left ventricle 124. Generally, the annulus of the aortic valve 140 is substantially circular or cylindrical, while the annulus of the mitral valve 130 is substantially elliptical. Blood flows through heart 100 in the direction shown by arrows "B".

An arrow labeled "TA" indicates a transapical approach of implanting a prosthetic heart valve, in this case to replace the mitral valve 130 of a patient. In transapical delivery, a small incision is made between the ribs and into the apex of the left ventricle 124 to deliver the prosthetic heart valve to the target site.

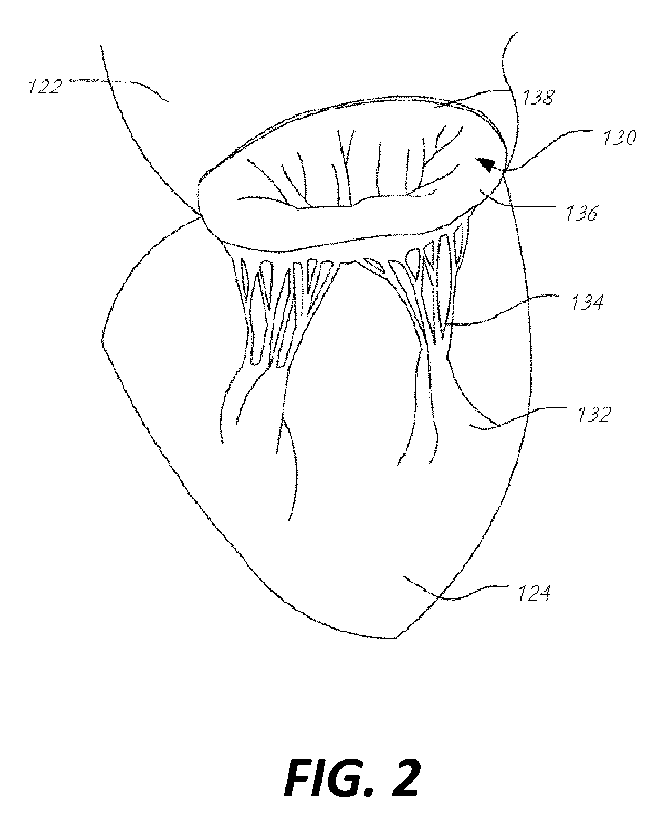

FIG.2 is a more detailed schematic representation of native mitral valve 130 and its associated structures. As previously noted, mitral valve 130 includes two flaps or leaflets, a posterior leaflet 136 and an anterior leaflet 138, disposed between left atrium 122 and left ventricle 124. Cord-like tendons known as chordae tendineae 134 connect the two leaflets 136, 138 to the medial and lateral papillary muscles 132. During atrial systole, blood flows down the pressure gradient from the left atrium 122 to the left ventricle 124. When the left ventricle 124 contracts in ventricular systole, the increased blood pressure in the chamber pushes the leaflets 136 and 138 of the mitral valve 130 to close, preventing backflow of blood into the left atrium 122. Since the blood pressure in the left atrium 122 is much lower than that in the left ventricle 124, the leaflets attempt to evert to the low pressure regions. The chordae tendineae 134 prevent the eversion by becoming tense, thus pulling on the leaflets and holding them in the closed position.

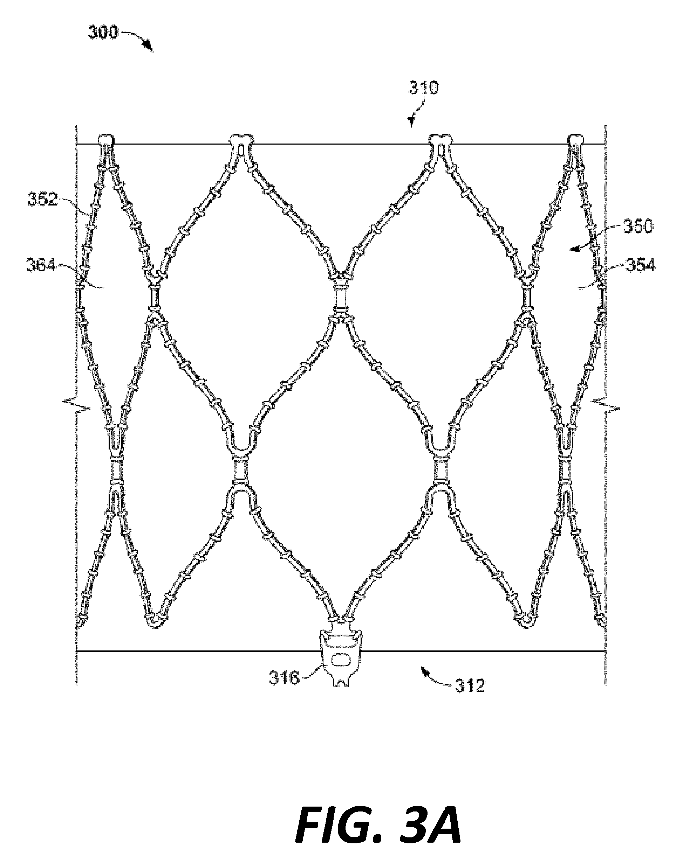

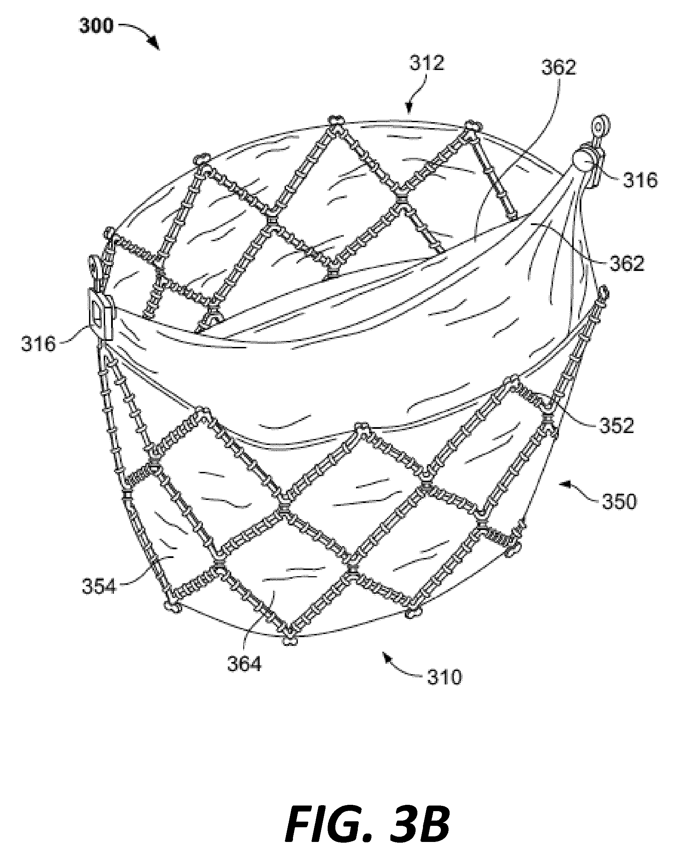

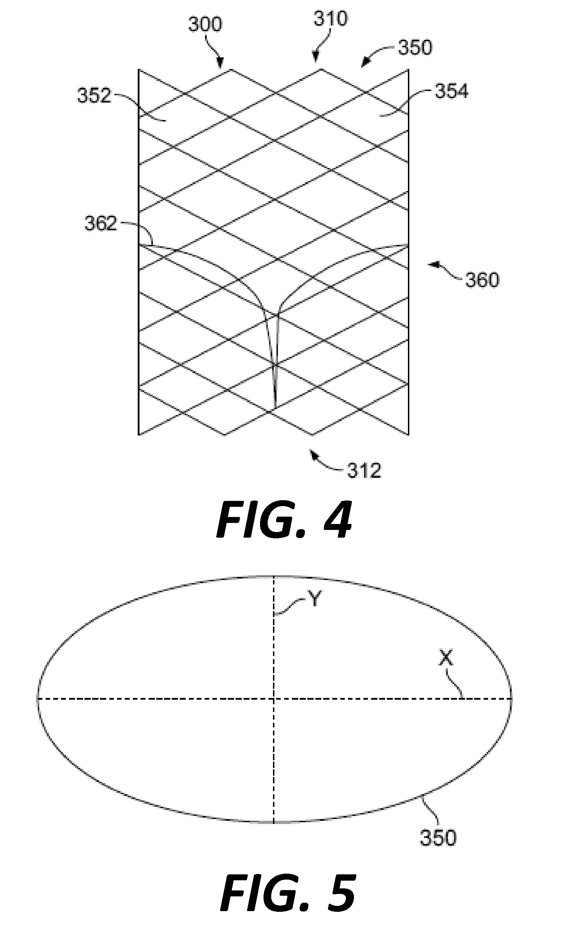

FIGS.3A, 3B and 4 are side, perspective, and longitudinal cross-sectional views of a prosthetic heart valve 300 according to an embodiment of the disclosure. Prosthetic heart valve 300 is a collapsible prosthetic heart valve designed to replace the function of the native mitral valve of a patient (see native mitral valve 130 of FIGS. 1-2). Generally, prosthetic valve 300 has an inflow end 310 and outflow end 312. When used to replace native mitral valve 130, prosthetic valve 300 may have a low profile so as not to interfere with atrial function in the native valve annulus. It should be understood that the orientation of prosthetic valve 300 in FIG. 3A is substantially opposite than in FIG. 3B.

Prosthetic heart valve 300 may include stent 350, which may be formed from biocompatible materials that are capable of self-expansion, such as shape memory alloys including Nitinol. Stent 350 may include a plurality of struts 352 that form cells 354 connected to one another in one or more annular rows around the stent. Cells 354 may all be of substantially the same size around the perimeter and along the length of stent 350. Alternatively, the cells 354 near inflow end 310 may be larger than the cells near outflow end 312. Stent 350 may be radially expandable to provide a radial force to assist with positioning and stabilizing prosthetic heart valve 300 in the native valve annulus.

It is preferable that stent 350 have a substantially elliptical shape or generally a "D"-shape. FIG. 5 is an end view of stent 350 of prosthetic heart valve 300 in a fully expanded condition, with the remaining components of prosthetic heart valve 300 omitted for clarity. In this example, stent 350 is substantially elliptical with a major axis X and a minor axis Y. The ratio of the length of the minor axis Y to the length of the major axis X may be between about 0.7 and 0.99. However, other ranges including between about 0.7 and about 0.9, between about 0.7 about 0.8, and between about 0.7 and about 0.75 may be suitable. Two commissure attachment features 316 may be positioned at diametrically opposed sides of the stent 350 along the major axis X. As noted above, a stent with a substantially cylindrical shape in the expanded condition may be substantially deformed upon implantation into native the mitral valve annulus. The substantially elliptical shape of stent 350, particularly when ratios of the length of the minor axis Y to the length of the major axis X are in the ranges described above, undergoes less deformation when implanted in the mitral valve annulus. With less deformation of the shape of implanted stent 350, the valve leaflets, described in greater detail below, coapt with greater fidelity, resulting in less regurgitation.

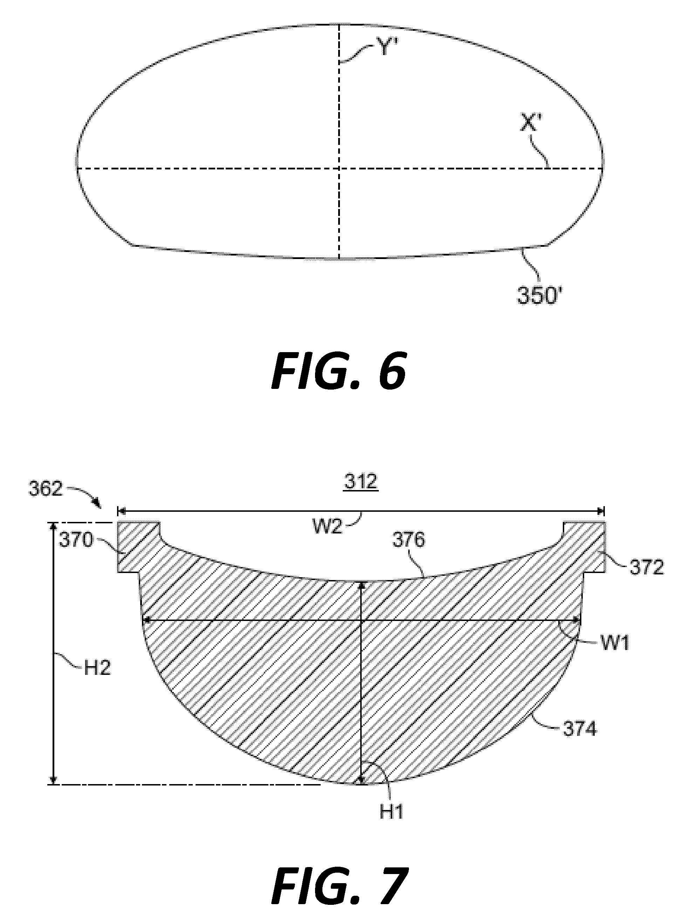

Alternately, as shown in FIG. 6, a modified stent 350' may be substantially "D"-shaped. In other words, stent 350' may take the form of an ellipse with one of the long sides substantially flattened, such that major axis X' may be substantially similar in length to major axis X of stent 350, while minor axis Y' of stent 350' is shorter in length than minor axis Y of stent 350. In addition to undergoing less deformation than a more cylindrical stent, the flat side of stent 350', when implanted facing aortic valve 140, may exert relatively little radial force on aortic valve 140 through the heart wall separating mitral valve 130 from aortic valve 140, in turn resulting in little or no disruption of the function of aortic valve 140. The potential disruption of the function of aortic valve 140 is described in greater detail below.

Prosthetic heart valve 300 may also include a valve assembly 360 including a pair of leaflets 362 (FIGS. 3B, 4) attached to a cuff 364 (FIGS. 3A, 3B). Valve assembly 360 may be attached to struts 352 of stent 350. In FIG. 3A, cuff 364 is illustrated as being disposed on the lumenal or inner surface of stent 350. It should be noted that cuff 364 may alternately be disposed on the ablumenal or outer surface of stent 350, or on both the lumenal and ablumenal surfaces, for example by being wrapped around an end of stent 350. Leaflets 362 replace the function of native mitral valve leaflets 136 and 138 described above with reference to FIG. 2. That is, leaflets 362 coapt with one another to function as a one-way valve. Prosthetic heart valve 300 is illustrated as having a valve assembly 360 with two leaflets 362. Both cuff 364 and leaflets 362 may be wholly or partly formed from any suitable biological material, such as bovine or porcine pericardium, or from one or more polymers, such as polytetrafluoroethylene (PTFE), urethanes and the like. Valve assembly 360 may be secured to stent 350 by suturing to struts 352 or by using tissue glue, ultrasonic welding or other suitable methods.

One leaflet 362 of prosthetic valve 300 is illustrated in a flattened configuration in FIG. 7. Generally, leaflet 362 includes a first edge 374 having a generally arcuate shape and a second or free edge 376 having a less pronounced arcuate shape. A first end of first edge 374 may be connected to a first end of second edge 376 by first tab 370, which may be at least partially rectangular and provide a surface for attachment to stent 350, described in greater detail below. Similarly, a second end of first edge 374 may be connected to a second end of second edge 376 by second tab 372, which may be substantially identical to first tab 370.

The shape and size of the leaflets 362 also may have a significant bearing on the effectiveness of prosthetic valve 300. In the illustrated embodiment, leaflet 362 has a first height H1 measured vertically (with reference to FIG. 7) from the midpoint or valley of first edge 374 to the midpoint or valley of second edge 376, and a second height H2 measured vertically (with reference to FIG. 7) from the midpoint or valley of first edge 374 to the farthest edge of first tab 370. The leaflet 362 also has a first width W1 measured substantially horizontally (with reference to FIG. 7) from the junction of the first edge 374 with the first tab 370 to the junction of the first edge 374 with the second tab 372, and a second width W2 measured substantially horizontally (with reference to FIG. 7) from an outer edge of first tab 370 to an outer edge of second tab 372. It should be understood that tabs 370 and 372 may be folded as illustrated in FIG. 7 in preparation for attachment to stent 350.

In one example, the ratio of height H1 to width W1 of each leaflet 362 is about 0.46. In other examples, the ratio of height H1 to width W1 may be between about 0.4 and about 0.5, although the ratio may be as large as about 0.65. The above examples may be particularly useful in that the same ratio of height H1 to width W1 may be used for patients with differently sized mitral valve annuli, regardless of the absolute size. Thus, a patient having a relatively small mitral valve annulus and a patient having a relatively large mitral valve annulus may both be treated with a prosthetic heart valve 300 having leaflets 362 with a height H1 to width W1 ratio of about 0.46, even if the absolute size of the leaflets differs significantly. For example, patients may have mitral valve annuli ranging in size from about 29 mm to about 51 mm. In one example for a relatively small annulus size of 31 mm, each leaflet 362 of the prosthetic heart valve 300 may have a height H1 of about 20 mm, a height H2 of about 25.8 mm, a width W1 of about 43.5 mm, and a width W2 of about 48.75 mm.

The first edge 374 of each leaflet 362 may be attached to the cuff 364 (and to stent 350 if desired) between two commissure attachment features 316 of stent 350 by any suitable attachment means, such as suturing, stapling, adhesives or bonding via laser, ultrasound or heat, as well as by any other suitable method. For example, the first edge 374 of each leaflet 362 may be sutured to the cuff 364 and stent 350 by passing strings or sutures through the cuff 364 of the valve assembly 360 and around struts 352. The leaflets 362 may be attached to the stent 350 along at least some struts 352 and through the eyelets in commissure attachment features 316 to enhance the structural integrity of the valve assembly 360. The second or free edge 376 of each leaflet 362 may coapt with the corresponding free edge of the other leaflet, thereby enabling the leaflets to function collectively as a one-way valve. Bi-leaflet valves generally include two commissure attachment features 316, with only one being visible in FIG. 3A.

In prosthetic heart valve 300, the first tab 370 of leaflet 362 is attached to one commissure attachment feature 316, with the second tab 372 being attached to the other commissure attachment feature 316. A second leaflet substantially identical to leaflet 362 is similarly attached, so that one tab of each leaflet is attached to one commissure attachment feature and the other tab of each leaflet is attached to the other commissure attachment feature. In this configuration, the second or free edge 376 of each leaflet 362 is adjacent the outflow end 312 of stent 350, with the first edge 374 of each leaflet 362 being closer to the inflow end 310 of stent 350. It should be noted that the prosthetic heart valve 300 in FIGS. 3A-4 is oriented with outflow end 312 pointing downward, similar to the orientation of the prosthetic heart valve 300 after implantation into a native mitral valve annulus. On the other hand, leaflet 362 in FIG. 7 is illustrated in a conventional manner with a substantially opposite orientation. In use, first and second tabs 370 and 372 align with the commissure attachment features 316 of stent 350.

In order to implant prosthetic heart valve 300 into the native mitral valve annulus of a patient, the valve 300, including stent 350 and valve assembly 360, may be crimped down to a collapsed condition, loaded into a delivery device (not shown), and covered by a sheath of the delivery device to maintain the valve in the collapsed condition. The delivery device is advanced to the native mitral valve annulus, for example through the vasculature via an opening in the femoral artery

(transfemoral delivery), or through an incision in the apex of left ventricle 124 (transapical delivery). Other delivery methods, such as transseptal delivery, are also contemplated herein. Once the sheath is positioned at the desired location with respect to the native mitral valve annulus, which may be confirmed by imaging techniques such as fluoroscopy, the sheath may be advanced or retracted relative to the remainder of the delivery device. As the sheath is moved from around prosthetic heart valve 300, constrictive forces are removed from the valve, which begins to expand as stent 350 begins to return to its set shape (*i.e.,* the expanded condition). As noted above, upon implantation, the inflow end 310 of stent 350 and the first edge 374 of each leaflet 362 are oriented toward left atrium 122 while the outflow end 312 of stent 350 and the second or free edge 376 of each leaflet 362 are oriented toward left ventricle 124.

Because of the elliptical shape of the annulus of native mitral valve 130, it is important that prosthetic heart valve 300, once released from the delivery device, properly aligns with the mitral valve annulus. In other words, the major axis X of stent 350 should align with the major axis of the native mitral valve annulus, while the minor axis Y of stent 350 should align with the minor axis of the native mitral valve annulus. Axes of the mitral valve annulus are not identified in the figures. Proper alignment reduces the deformation of the prosthetic heart valve 300 by the native anatomy, allowing leaflets 362 to operate and coapt with one another as intended with minimal regurgitation through prosthetic valve 300. Further, because the mitral valve 130 and aortic valve 140 are separated by a thin wall, forces exerted on the mitral valve annulus, particularly those in the direction of aortic valve 140, may interrupt proper functioning of aortic valve 140. Because aortic valve 140 controls blood flow to the aorta 110, which is the main artery in the body, it is important to minimize interference with the proper function of aortic valve 140. The force exerted upon the thin wall separating mitral valve 130 and aortic valve 140 may be even further reduced if "D"-shaped stent 350' is used to form the prosthetic mitral valve. If using stent 350', the flattened portion of the "D" shape is preferably oriented toward the wall separating mitral valve 130 and aortic valve 140. In the fully expanded condition, the portion of stent 350' in contact with the wall separating the mitral valve 130 and aortic valve 140 exerts less radial force on the wall, and thus on the aortic valve 140, than a stent in which the portion contacting the wall has a greater degree of curvature.

As noted above, it is desirable that prosthetic valve 300, upon implantation, be oriented such that the major axis X of stent 350 substantially aligns with the major axis of the mitral valve annulus. In this orientation, the two commissure attachment features 316 of stent 350, which are positioned along major axis X, are not in contact with the portion of the heart wall adjacent aortic valve 140. Commissure attachment features are formed of a relatively large volume of material and are relatively stiff points compared to the remainder of the stent. The use of two commissure attachment features 316 along the major axis X of elliptical stent 350 allows for the orientation described above, which may help to further minimize the forces exerted by stent 350 on aortic valve 140 through the heart wall after implantation. In addition, the use of two commissure attachment features 316 allows for less stent material to protrude into the left ventricle 124 from the mitral valve annulus. When compared to prosthetic valve 300, the use of a prosthetic tri-leaflet valve having a stent with three commissure attachment features would increase the likelihood of interfering with aortic valve 140, and would result in a greater amount of stent material protruding into left ventricle 124, potentially interfering with the native anatomy.

In addition to the benefits of prosthetic heart valve 300 described above, the use of two leaflets 362 instead of three or more leaflets may require less total leaflet material, and therefore may reduce the complexity of the prosthetic heart valve 300. With only two leaflets 362, there are fewer opportunities for coaptation problems since only two surfaces are coapting. However, although a circular or cylindrical bi-leaflet valve may have the ability to coapt, it has been found that an elliptical bi-leaflet valve allows less regurgitation than a circular or cylindrical bi-leaflet valve. For example, testing indicates that at an average pressure differential of 3.7 mm Hg, a bi-leaflet valve with a circular cross-section may have an effective orifice area ("EOA") of approximately 3.28 cm², with approximately 14.5% regurgitation. In contrast, at the same average pressure differential of 3.7 mm Hg, a bi-leaflet valve with an elliptical cross-section may have an EOA of approximately 3.18 cm² but substantially reduced regurgitation of approximately 10.8%.

According to one embodiment of the disclosure, a prosthetic mitral valve comprises:
a collapsible and expandable stent;
first and second commissure attachment features disposed on the stent; and
a collapsible and expandable valve assembly disposed within the stent, the valve assembly including two leaflets, each leaflet having a first edge operably coupled to the stent, a second free edge, a first tab connecting a first end of the first edge to a first end of the second free edge, and a second tab connecting a second end of the first edge to a second end of the second free edge, each leaflet having a height measured from a midpoint of the first edge to a midpoint of the second edge, and a width measured from a junction of the first edge with the first tab to a junction of the first edge with the second tab;
wherein a ratio of the height to the width is between about 0.4 and about 0.65; and/or
the ratio of the height to the width is between about 0.4 and about 0.5; and/or
the ratio of the height to the width is about 0.46; and/or
the height is about 20 mm and the width is about 43.5 mm; and/or
the stent in an expanded condition is substantially elliptical in transverse cross-section; and/or
the cross-section of the stent in the expanded condition has a minor axis with a length and a major axis with a length, a ratio of the length of the minor axis to the length of the major axis being between about 0.7 and about 0.99; and/or
the first and second commissure attachment features are positioned at diametrically opposed positions on the stent substantially along the major axis; and/or
the stent in an expanded condition is substantially "D"-shaped.

In another embodiment of the disclosure, a prosthetic mitral valve comprises:
a collapsible and expandable stent, the stent in an expanded condition being substantially elliptical in transverse cross-section with a minor axis having a length and a major axis having a length, a ratio of the length of the minor axis to the length of the major axis being between about 0.7 and about 0.99;
first and second commissure attachment features disposed on the stent; and
a collapsible and expandable valve assembly disposed within the stent, the valve assembly including two leaflets, a first portion of each leaflet coupled to the first commissure attachment feature and a second portion of each leaflet coupled to the second commissure attachment feature; and/or
each leaflet has a first edge operably coupled to the stent, a second free edge, a first tab connecting a first end of the first edge to a first end of the second free edge, and a second tab connecting a second end of the first edge to a second end of the second free edge, each leaflet having a height measured from a midpoint of the first edge to a midpoint of the second edge, and a width measured from a junction of the first edge with the first tab to a junction of the first edge with the second tab, a ratio of the height to the width being between about 0.4 and about 0.65; and/or
the ratio of the height to the width is between about 0.4 and 0.5; and/or
the ratio of the height to the width is about 0.46; and/or
the height is about 20 mm and the width is about 43.5 mm; and/or
the first and second commissure attachment features are positioned at diametrically opposed positions on the stent substantially along the major axis.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims. For example, features of one embodiment of the invention may be combined with features of one or more other embodiments of the invention without departing from the scope of the invention.

## Claims

1. A prosthetic mitral valve, comprising:
a collapsible and expandable stent;
first and second commissure attachment features disposed on the stent; and
a collapsible and expandable valve assembly disposed within the stent, the valve assembly including two leaflets, each leaflet having a first edge operably coupled to the stent, a second free edge, a first tab connecting a first end of the first edge to a first end of the second free edge, and a second tab connecting a second end of the first edge to a second end of the second free edge, each leaflet having a height measured from a midpoint of the first edge to a midpoint of the second edge, and a width measured from a junction of the first edge with the first tab to a junction of the first edge with the second tab;
wherein a ratio of the height to the width is between about 0.4 and about 0.65.

2. The prosthetic mitral valve of claim 1, wherein the ratio of the height to the width is between about 0.4 and about 0.5.

3. The prosthetic mitral valve of claim 2, wherein the ratio of the height to the width is about 0.46.

4. The prosthetic mitral valve of claim 3, wherein the height is about 20 mm and the width is about 43.5 mm.

5. The prosthetic mitral valve of claim 1, wherein the stent in an expanded condition is substantially elliptical in transverse cross-section.

6. The prosthetic mitral valve of claim 5, wherein the cross-section of the stent in the expanded condition has a minor axis with a length and a major axis with a length, a ratio of the length of the minor axis to the length of the major axis being between about 0.7 and about 0.99.

7. The prosthetic mitral valve of claim 6, wherein the first and second commissure attachment features are positioned at diametrically opposed positions on the stent substantially along the major axis.

8. The prosthetic mitral valve of claim 1, wherein the stent in an expanded condition is substantially "D"-shaped.

9. A prosthetic mitral valve, comprising:
a collapsible and expandable stent, the stent in an expanded condition being substantially elliptical in transverse cross-section with a minor axis having a length and a major axis having a length, a ratio of the length of the minor axis to the length of the major axis being between about 0.7 and about 0.99;
first and second commissure attachment features disposed on the stent; and
a collapsible and expandable valve assembly disposed within the stent, the valve assembly including two leaflets, a first portion of each leaflet coupled to the first commissure attachment feature and a second portion of each leaflet coupled to the second commissure attachment feature.

10. The prosthetic mitral valve of claim 9, wherein each leaflet has a first edge operably coupled to the stent, a second free edge, a first tab connecting a first end of the first edge to a first end of the second free edge, and a second tab connecting a second end of the first edge to a second end of the second free edge, each leaflet having a height measured from a midpoint of the first edge to a midpoint of the second edge, and a width measured from a junction of the first edge with the first tab to a junction of the first edge with the second tab, a ratio of the height to the width being between about 0.4 and about 0.65.

11. The prosthetic mitral valve of claim 10, wherein the ratio of the height to the width is between about 0.4 and 0.5.

12. The prosthetic mitral valve of claim 11, wherein the ratio of the height to the width is about 0.46.

13. The prosthetic mitral valve of claim 12, wherein the height is about 20 mm and the width is about 43.5 mm.

14. The prosthetic valve of claim 9, wherein the first and second commissure attachment features are positioned at diametrically opposed positions on the stent substantially along the major axis.
